# EUROPÄISCHE PATENTANMELDUNG

(11) **EP 1 707 557 A1**
(43) Veröffentlichungstag der Anmeldung: **04.10.2006**
(21) Anmeldenummer: 06005727.0
(22) Anmeldetag: 21.03.2006
(51) Int. Cl.: C07C 209/78

(54) **Verfahren zur Herstellung von Di- und Polyaminen der Diphenylmethanreihe**

(30) Priorität: 30.03.2005 DE 102005014244
(71) Anmelder: Bayer MaterialScience AG, 51368 Leverkusen (DE)
(72) Erfinder: Hagen, Torsten Dr., 45257 Essen (DE); Grabowski, Stefan Dr., 41539 Dormagen (DE); Adamson, Richard, 42799 Leichlingen (DE); Koch, Daniel Dr., 47137 Duisburg (DE); Wershofen, Stefan Dr., 41065 Mönchengladbach (DE)

(57) **Zusammenfassung**

Die Erfindung betrifft ein Verfahren zur Herstellung von Di- und Polyaminen der Diphenylmethanreihe, bei dem man
a) eine erste Teilmenge an Anilin mit einem sauren Katalysator vermischt, und
b) eine zweite Teilmenge an Anilin mit Formaldehyd vermischt, und
c) die in den Schritt a) und b) hergestellten Mischungen anschließend miteinander vermischt und zur Reaktion bringt.

## Beschreibung

Die Erfindung betrifft ein Verfahren zur Herstellung von Di- und Polyaminen der Diphenylmethanreihe (MDA) durch Umsetzung von Anilin mit Formaldehyd in Gegenwart saurer Katalysatoren, bei dem man eine erste Teilmenge des Anilins mit dem sauren Katalysator vermischt, und eine zweite Teilmenge des Anilins mit Formaldehyd vermischt, und diese Mischungen anschließend miteinander vermischt und zur Reaktion bringt.

Die Herstellung von MDA ist allgemein bekannt und erfolgt üblicherweise durch Umsetzung von Anilin mit Formaldehyd in Gegenwart saurer Katalysatoren in einem kontinuierlichen, halbkontinuierlichen oder diskontinuierlichen Verfahren. Der Prozess ist in zahlreichen Patenten und Publikationen beschrieben (siehe z.B. H.J. Twitchett, Chem. Soc. Rev. 3(2), 209 (1974), M.V. Moore in: Kirk-Othmer Encycl. Chem. Technol., 3rd. Ed., New York, 2, 338-348 (1978)).

Unter Polyaminen der Diphenylmethanreihe (MDA) werden Amine und Gemische von Aminen folgenden Typs verstanden: 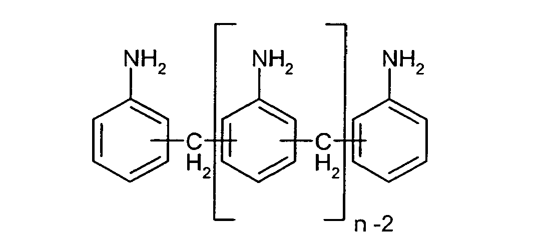 wobei n für eine natürliche Zahl ≥ 2 steht.

Bei der Umsetzung von Anilin und Formaldehyd entsteht üblicherweise ein Gemisch von Isomeren, weil die CH₂-Gruppe den aromatischen Ring des Anilins in 2-Position und/oder 4-Position und/oder 6-Position substituieren kann. Weiterhin entsteht bei dieser Reaktion ein Gemisch von Homologen, da n Moleküle Anilin mit (n-1) Molekülen Formaldehyd zu einem Polyamin der Kettenlänge n reagieren können. Die Zusammensetzung des Isomeren- und Homologengeinisches ist unterschiedlich in Abhängigkeit von den Reaktionsbedingungen und der verwendeten Rezeptur. Im allgemeinen ist ein hoher Gehalt an 4,4'-MDA (n = 2) im Reaktionsprodukt erwünscht.

Es sind eine Reihe von Methoden bekannt, um die Selektivität zu Gunsten von 4,4`-MDA zu steigern. Zum Beispiel führen eine Steigerung der spezifischen Konzentration an saurem Katalysator im Reaktionsgemisch, eine Erhöhung des Anilinüberschusses und niedrige Reaktionstemperaturen zu einer Steigerung der Ausbeute an 4,4'-MDA. So beschreibt z.B. DE-A-1 643 449 die Herstellung von MDA mit einem hohen Anteil an 4,4'-MDA durch Umsetzung von zuvor mit Säure umgesetztem Anilin mit Formaldehyd, wobei der Protonierungsgrad mindestens 25 %, vorzugsweise mindestens 50 %, und noch besser 75 - 100 % beträgt. DE-A-10 111 337 beschreibt die Herstellung von MDA bei einem Protonierungsgrad von < 20 %, wobei allerdings erfindungsgemäß MDA mit einem erhöhten Anteil an 2,4'-Isomeren erhalten wird. In EP-A-10 53 222 wird beschrieben, dass eine hohe Temperatur zu MDA mit einem erhöhten Anteil an 2,4'-und 2,2'-Isomeren führt.

Alle bekannten Maßnahmen zur Steigerung der Selektivität zu Gunsten von 4,4`-MDA haben jedoch den Nachteil, dass die Wirtschaftlichkeit des Verfahrens geringer wird. Eine höhere spezifische Einsatzmenge an saurem Katalysator steigert die Materialkosten, da der Katalysator in der Aufarbeitung durch Neutralisation entfernt und damit verbraucht wird. Die überschüssige Anilinmenge senkt die Raum-Zeit-Ausbeute und erhöht den Energieverbrauch bei der Rückdestillation. Niedrigere Reaktionstemperaturen erfordern längere Reaktionszeiten, um den Umsatz zu vervollständigen.

Eine wesentliche Schwierigkeit insbesondere bei der technischen Ausführung dieses Prozesses besteht darin, dass die ablaufenden chemischen Reaktionen stark exotherm und die selektivitätsbestimmenden Reaktionsschritte sehr schnell sind (H.-J. Ladwig, W. Pippel, C. Ringel, H. Oelmann, "Einfaches kinetisches Modell der Anilin-Formaldehyd-Kondensation", Wissenschaftliche Zeitschrift der TU Dresden, 38, 1989, S. 121 - 126). Das führt dazu, dass diese Reaktionen zumindest teilweise bereits während der Durchmischung der Reaktionspartner ablaufen und dabei lokale Temperatur- bzw. Konzentrationsgradienten auftreten können, die die Selektivität vermindern. Zum Beispiel fiihren erhöhte Temperaturen bei der Vermischung von Formaldehyd mit einem Reaktionsgemisch, das Anilin und sauren Katalysator enthält, zu einer Erhöhung des Anteils an höheren Oligomeren bzw. ortho-Isomeren. Die bislang bekannten Methoden zur Unterdrückung von Selektivitätseinbußen auf Grund von Temperaturspitzen bei der Vermischung sind jedoch nicht ausreichend wirkungsvoll.

Die Aufgabe der vorliegenden Erfindung bestand deshalb darin, ein einfaches und wirtschaftliches Verfahren zur Herstellung von MDA bereit zu stellen, bei dem die Bildung unerwünschter Isomeren, Homologen oder Nebenprodukte vermindert bzw. unterdrückt ist.

Die Erfindung betrifft ein Verfahren zur Herstellung von Di- und Polyaminen der Diphenylmethanreihe, bei dem man
a) eine erste Teilmenge an Anilin mit einem sauren Katalysator vermischt, und
b) eine zweite Teilmenge an Anilin mit Formaldehyd vermischt, und
c) die in den Schritt a) und b) hergestellten Mischungen anschließend miteinander vermischt und zur Reaktion bringt.

Der Vorteil des erfindungsgemäßen Verfahrens besteht darin, dass durch die Aufteilung der Vermischung in die drei Schritte a), b) und c) die insgesamt entstehende Misch- und Reaktionswärme ebenfalls auf diese drei Schritte verteilt anfällt. Die in jedem der einzelnen Teilschritte a), b) und c) anfallende Misch- und Reaktionswärme ist somit relativ geringer als bei einem Verfahren, bei dem diese drei Schritte nicht in der beschriebenen Weise getrennt durchgeführt werden.

Dies ist insbesondere bei großen Durchsätzen an in Schritt c) erzeugten Mischungen von bevorzugt größer als 5 m³/h, besonders bevorzugt größer als 10 m³/h bzw. bei Verwendung großer Apparate für die Durchführung der Vermischung und / oder der Reaktion in Schritt c) mit einem Volumen der Apparate von größer 3 m³, besonders bevorzugt von größer 5 m³, von Vorteil, weil bei solchen großen Apparaten das Verhältnis von Oberfläche zu Volumen im Allgemeinen reduziert und daher die Kühlleistung limitiert ist und eine ausreichende externe Kühlung somit nur mit großem technischen Aufwand realisierbar ist.

Die Kühlung kann dabei so erfolgen, dass die Reaktionswärme über die Außenwand des Reaktors durch einen Kühlmantel abgeführt wird. Als Kühlmedium wird bevorzugt Kühlwasser mit einer Temperatur von 2 - 45°C, besonders bevorzugt 20 - 35°C eingesetzt. Es ist auch möglich, Reaktoren mit Umpumpkreislauf und darin integriertem Wärmeaustauscher zu verwenden. Weiterhin ist es möglich, Reaktoren mit Vakuumsystem und darin integriertem Wärmetauscher (Siedekühlung) zu verwenden. Es ist möglich, eine dieser Kühlmöglichkeiten oder Kombinationen aus diesen Kühlmöglichkeiten zu nutzen. Die insgesamt zur Verfügung stehende Kühlleistung ist dabei aus den Kühlleistungen durch die Wandflächen des Reaktors, des im Kreislauf eingebauten Wärmeaustauschers und des im Brüdensystem eingebauten Wärmeaustauschers zusammengesetzt.

Insbesondere ist es aber von Vorteil, dass in Schritt a) und b) keine selektivitätsbestimmenden Reaktionen ablaufen. Die infolge der freiwerdenden Reaktionswärme in Schritt a) und b) ggf. auftretende Temperaturerhöhung führt also nicht zu einer Verminderung der Selektivität. Die Reaktionswärme aus Schritt a) und b) kann z.B. durch Kühlung abgeführt werden und die aus a) und b) entstehenden Gemische können bei niedriger Temperatur der Vermischung in Schritt c) zugeführt werden.

Geeignete Temperaturen für die Vermischung in Schritt a) liegen im Bereich von 0 bis 100°C, bevorzugt im Bereich von 20 bis 95°C. Geeignete Temperaturen für die Vermischung in Schritt b) liegen im Bereich von 20 bis 100°C, bevorzugt von 40 bis 100°C, besonders bevorzugt von 60 bis 95°C. Die selektivitätsbestimmenden Reaktionen laufen erst während oder nach der Vermischung in Schritt c) ab. Geeignete Temperaturen für die Vermischung in Schritt c) liegen im Bereich von 20°C bis 250°C. Bevorzugt wird dabei so vorgegangen, dass während der Vermischung in Schritt c) die Temperatur im Bereich von 20 bis 100°C, bevorzugt von 20 bis 80°C, besonders bevorzugt von 20 bis 60°C gehalten wird. Dies kann durch externe Kühlung und/oder durch die Einstellung der Temperatur der eingesetzten, vorher in den Schritten a) und b) erhaltenen Mischungen erreicht werden. Bevorzugt wird die in Schritt a) erhaltene Mischung mit einer Temperatur von 15 bis 100°C, besonders bevorzugt von 20 bis 60°C eingesetzt. Bevorzugt wird die in Schritt b) erhaltene Mischung mit einer Temperatur von 40 bis 130°C, besonders bevorzugt von 50 bis 100°C eingesetzt. Danach wird die Temperatur, ggf. nach einer Vorreaktionszeit von 0 bis 100 min bei einer Temperatur von 20 bis 100°C, bevorzugt 30 bis 95°C, kontinuierlich oder in Stufen auf eine Endtemperatur von 90 bis 250 °C, bevorzugt 100 bis 160°C gesteigert.

Das erfindungsgemäße Verfahren wird besonders vorteilhaft in großem Maßstab wie weiter oben beschrieben durchgeführt, da die erforderlichen Einrichtungen zur Entfernung der Wärme weniger aufwendig sein können. Die erforderliche Kühlleistung liegt dabei für die Summe der Schritte a), b) und c) bevorzugt bei > 500kW, besonders bevorzugt bei > 1000 kW. Da die nicht selektivitätsbestimmenden Schritte a) und b) bevorzugt bei 20 bis 95°C bzw. 60 bis 95°C durchgeführt werden, der selektivitätsbestimmende Schritt c) jedoch bei tieferer Temperatur von bevorzugt 20 bis 60°C durchgeführt wird, ergibt sich bei einer gegebenen Kühlwassertemperatur von 20 bis 35°C für die Schritte a) und b) eine größere Temperaturdifferenz. Dadurch ist für eine bestimmte Kühlleistung entsprechend ein Wärmetauscher mit kleinerer Wärmetauscherfläche ausreichend. Deshalb ist im erfindungsgemäßen Verfahren die Summe der erforderlichen Wärmetauscherfläche für die Schritte a), b) und c) kleiner als bei einem Verfahren, bei dem diese drei Schritte nicht in der beschriebenen Weise getrennt durchgeführt werden und also die gesamte Wärme aus Selektivitätsgründen bei tieferer Temperatur und bei kleinerer Temperaturdifferenz zum Kühlmedium entfernt werden muss.

Die gesamte Reaktionsdauer liegt üblicherweise zwischen 30 und 750 min, bevorzugt von 50 bis 300 min, besonders bevorzugt von 90 bis 180 min. Der wesentliche Vorteil des erfindungsgemäßen Verfahrens besteht darin, dass die in Schritt c) freiwerdende restliche Reaktionswärme nur ein Teil der Gesamt-Reaktionswärme darstellt, da der andere Teil der Reaktionswärme bereits in den nicht selektivitätsbestimmenden Schritten a) und b) frei gesetzt worden ist. Somit werden Nebenreaktionen infolge von Temperaturspitzen beim Vermischen wirkungsvoll unterdrückt. Auch dieser Effekt ist besonders vorteilhaft bei der Durchfiihrung des Verfahrens in großem Maßstab, wie weiter oben beschrieben, da die zur Vermischung eingesetzten Apparate weniger aufwendig sein können.

Das Verfahren kann generell kontinuierlich, halbkontinuierlich oder diskontinuierlich durchgeführt werden. Geeignete Apparate für die Durchführung der Reaktion in Schritt c) sind zum Beispiel gerührte Reaktoren, Rohrreaktoren oder auch Rohrreaktoren mit Einbauten wie Lochböden, welche die Verweilzeitcharakteristik im Reaktor beeinflussen. Geeignet ist auch eine Kombination aus mehreren Reaktortypen. Der absolute Reaktionsdruck liegt üblicherweise im Bereich von 0,01 bar bis 10 bar, bevorzugt im Bereich von 0,03 bar bis 5 bar.

Geeignete saure Katalysatoren sind starke organische oder anorganische Säuren, beispielsweise Salzsäure, Schwefelsäure, Phosphorsäure, Methansulfonsäure oder feste Säuren wie z.B. Zeolithe. Bevorzugt wird Salzsäure eingesetzt.

Die Konzentrationen und Konzentrationsverhältnisse der Ausgangsverbindungen sowie der Hilfsstoffe können im Prinzip frei gewählt werden. Üblicherweise wird Anilin als technische Ware mit einem Gehalt von 85 bis >99 Gew.-%, Formaldehyd als wässrige Lösung mit einem Gehalt von 20 bis 50 Gew.-% und Salzsäure als wässrige Lösung mit einem Gehalt von 20 bis 37 Gew.-% eingesetzt. Es ist aber ebenfalls möglich, wässrige Formaldehydlösungen anderer Konzentration oder andere Methylengruppen liefernde Verbindungen, wie z.B. Polyoxymethylenglykol, paraFormaldehyd oder Trioxan einzusetzen. Üblicherweise wird Anilin im Überschuß gegenüber Formaldehyd eingesetzt, wobei der molare Überschuss von Anilin im Bereich von 1,5 bis 10, bevorzugt 1,8 bis 5 beträgt. Die molare Menge an Salzsäure liegt üblicherweise im Bereich von 1 bis 50 % bezogen auf die eingesetzte molare Menge an Anilin, bevorzugt 5 bis 30 %. Die Aufteilung der Anilinmenge auf die Schritte a) und b) ist im Prinzip beliebig. Aus praktischen Erwägungen ist jedoch eine Konzentrationsfällung von Aniliniumhydrochlorid bei der gewünschten Reaktionstemperatur nach Möglichkeit zu vermeiden. Ebenso schränkt im Schritt b) die mit sinkendem Anilin:Formaldehyd-Verhältnis steigende Viskosität des Gemisches b) den Arbeitsbereich ein. Zur Herstellung bestimmter Produkttypen kann es deshalb vorteilhaft sein, neben der Formaldehydmenge, die im Schritt b) mit Anilin vermischt wird, der Reaktionsmischung nach der Vermischung in Schritt c) eine zusätzliche Menge Formaldehyd zuzugeben.

Bevorzugt wird daher Anilin im Verhältnis von 1:1 bis 1:9 auf die Schritte a) und b) verteilt eingesetzt.

Das in Schritt b) hergestellte Gemisch ist in der Regel zweiphasig. Die Wasserphase kann in einem zusätzlichen Trennschritt abgetrennt oder im Gemisch belassen werden. Bevorzugt wird das Wasser, z.B. durch Phasentrennung abgetrennt.

Die Reaktion kann in Substanz oder in einem Lösemittel durchgeführt werden. Geeignete Lösungsmittel sind beispielsweise Wasser, Alkohole, substituierte oder unsubstituierte aromatische Kohlenwasserstoffe. Bevorzugt wird ohne Zusatz eines Lösungsmittel gearbeitet.

Es ist weiterhin möglich, nach der Vermischung in Schritt c) zusätzlich Anilin, Formalin oder sauren Katalysator zuzusetzen.

Zur Aufarbeitung des in Schritt c) erhaltenen sauren Reaktionsgemisches wird das Reaktionsgemisch üblicherweise mit einer Base gemäß dem Stand der Technik neutralisiert. Nach dem Stand der Technik erfolgt die Neutralisation üblicherweise bei Temperaturen von beispielsweise 90 bis 100°C ohne Zusatz weiterer Substanzen. (H.J. Twitchett, Chem. Soc. Rev. 3(2), 223 (1974)). Sie kann aber auch auf einem anderen Temperaturniveau erfolgen, um z.B. den Abbau von störenden Nebenprodukten zu beschleunigen. Als Basen sind beispielsweise geeignet die Hydroxide der Alkali- und Erdalkalielemente. Vorzugsweise kommt wässrige NaOH zur Anwendung.

Die zur Neutralisation eingesetzte Base wird dabei bevorzugt in Mengen von größer 100%, besonders bevorzugt 105 bis 120% der für die Neutralisation des eingesetzten sauren Katalysators stöchiometrisch benötigten Menge eingesetzt.

Im Anschluss an die Neutralisation wird in einem Trennbehälter üblicherweise die organische von der wässrigen Phase getrennt. Die nach Abtrennung der wässrigen Phase verbleibende produktenthaltende organische Phase wird bevorzugt weiteren Aufarbeitungsschritten unterzogen (z.B. Wäsche) und anschließend von überschüssigem Anilin und anderen im Gemisch vorhandenen Stoffen (z.B. weiteren Lösungsmitteln) durch geeignete Verfahren wie z.B. Destillation, Extraktion oder Kristallisation befreit.

Die Erfindung betrifft auch ein Verfahren zur Herstellung von Di- und Polyisocyanaten der Diphenylmethanreihe, bei dem man
a) eine erste Teilmenge an Anilin mit einem sauren Katalysator vermischt, und
b) eine zweite Teilmenge an Anilin mit Formaldehyd vermischt, und
c) die in den Schritt a) und b) hergestellten Mischungen anschließend miteinander vermischt und zu Di - und Polyaminen der Diphenylmethanreihe umsetzt, und anschließend
d) die Di - und Polyamine der Diphenylmethanreihe mit Phosgen zu Di - und Polyisocyanaten der Diphenylmethanreihe umsetzt.

Dazu wird das nach dem erfindungsgemäßen Verfahren hergestellte MDA anschließend nach den bekannten Methoden mit Phosgen in einem inerten organischen Lösungsmittel zu den entsprechenden Isocyanaten umgesetzt. Das Molverhältnis von Roh-MDA zu Phosgen wird zweckmäßigerweise so bemessen, dass pro Mol NH₂-Gruppe 1 bis 10 Mol, vorzugsweise 1,3 bis 4 Mol Phosgen in der Reaktionsmischung vorliegen. Als inerte Lösungsmittel haben sich chlorierte, aromatische Kohlenwasserstoffe wie z.B. Monochlorbenzol, Dichlorbenzole, Trichlorbenzole, die entsprechenden Toluole und Xylole sowie Chlorethylbenzol bewährt. Insbesondere Anwendung finden als inerte organische Lösungsmittel Monochlorbenzol, Dichlorbenzol oder Mischungen dieser Chlorbenzole. Die Menge an Lösungsmittel wird zweckmäßigerweise so bemessen, dass die Reaktionsmischung einen Isocyanatgehalt von 2 bis 40 Gew.-%, vorzugsweise zwischen 5 und 20 Gew.-%, bezogen auf das Gesamtgewicht der Reaktionsmischung, aufweist. Nach beendeter Phosgenierung wird der Reaktionsmischung das überschüssige Phosgen, das inerte organische Lösungsmittel oder Mischungen davon durch Destillation abgetrennt.

Aus dem gewonnenen Roh-MDI können die gemäß dem Stand der Technik bekannten Produkte der polymeren MDI-Reihe, enthaltend zwei- und höherkernige Di- und Polyisocyanate der Diphenylmethanreihe, sowie der monomeren MDI-Reihe, enthaltend zweikernige Diisocyanate der Diphenylmethanreihe, hergestellt werden, insbesondere hochviskose polymere MDI-Typen mit 80 bis 3.000 mPas bei 25°C, technisch reines 4,4'-MDI und/oder technisch reines 2,4'-MDI sowie deren Mischformen. Die Abtrennung dieser Produkte aus dem Roh-MDI kann durch Verfahren gemäß dem Stand der Technik, beispielsweise Destillation, erfolgen. Diese Produkte eignen sich für den Einsatz als Rohstoffe für die Polyurethan-Herstellung in Form von Polymeren und Prepolymeren durch Reaktion mit Polyolen.

### Beispiele:

### Allgemeine Versuchsbeschreibung:

In einer Labor-Glasapparatur, ausgestattet mit Rührwerk und einem Kühlbad mit Eiswasser werden folgende Einsatzstoffe miteinander vermischt.

| | **Gehalt** | **Einwaage** | **Stoffmenge** |
|---|---|---|---|
| Anilin | > 99.9 Gew.-% | 465,7 g | 5,00 mol |
| Formaldehyd-Lösung | 30,12 Gew.% (HCHO) | 178,0 g | 1,79 mol |
| Salzsäure | 32,74 Gew.% (HCl) | 111,5 g | 1,00 mol |

Nach Abschluss der in den einzelnen Beispielen beschriebenen Vermischung beträgt die Temperatur des Gemisches 50°C. Dann wird das Kühlbad durch einen elektrischen Heizmantel ersetzt und innerhalb von 10 min auf 75°C aufgeheizt. Die Mischung wird 30 min bei 75°C gerührt und anschließend innerhalb von 30 min zum Sieden unter Rückfluss erhitzt. Die Innentemperatur beträgt 103 bis 105°C. Nach 10 Stunden unter Rückfluss werden 148,6 g 32,3%ige Natronlauge und 133 ml dest. Wasser zugegeben. Dabei entsteht eine zweiphasige Mischung, die 15 min intensiv verrührt wird. Danach werden die Phasen getrennt und die organische Phase noch zweimal mit je 400 mL dest. Wasser extrahiert. Aus der organischen Phase werden überschüssiges Anilin und Wasserreste durch Destillation im Vakuum (0,1 mbar) abgetrennt. Man erhält das Produkt MDA als Sumpf der Destillation. Dessen Zusammensetzung wird durch Umkehrphasen-HPLC bestimmt.

### Beschreibung der Vermischung:

### Beispiel 1 (Vergleichsbeispiel): Semi-Batch-Fahrweise: Zulauf Formaldehyd-Lösung zur Vorlage Anilin-hydrochlorid

In der Versuchsapparatur wird das Anilin (25°C) vorgelegt. Dazu wird unter Rühren die Salzsäure (25°C) zugegeben. Dabei entsteht eine klare Lösung. Die Temperatur steigt dabei auf 53°C an. Es wird auf 50°C temperiert. Dann wird innerhalb von 10 min unter Rühren die Formaldehyd-Lösung (25°C) zugetropft und anschließend noch 5 min nachgerührt. Während der Dosierung wird mit einem Eiswasserbad gekühlt. Die Innentemperatur steigt vorübergehend auf 51,0°C an.

### Beispiel 2 (Vergleichsbeispiel): Simultanzulauf Formaldehyd + HCl zu vorgelegtem Anilin

In der Versuchsapparatur wird das Anilin vorgelegt und auf 50°C temperiert. Dazu wird innerhalb von 10 min unter Rühren die Salzsäure (25°C) und die Formaldehyd-Lösung (25°C) gleichzeitig mit konstanter Geschwindigkeit zugetropft und anschließend noch 5 min nachgerührt. Während der Dosierung wird mit einem Eiswasserbad gekühlt. Die Innentemperatur steigt vorübergehend auf 61,5°C an.

### Beispiel 3 (Vergleichsbeispiel): Semi-Batch-Fahrweise: HCI-Zulauf zur Vorlage Aminal

### Variante I:

Das Anilin und die Formaldehyd-Lösung werden unter Rühren miteinander vermischt. Dabei entstehen zwei Phasen, die voneinander getrennt werden. Die organische Phase wird in der Versuchsapparatur vorgelegt und auf 50°C temperiert. Dazu wird innerhalb von 15 min unter Rühren die Salzsäure (25°C) zugetropft und anschließend noch 10 min nachgerührt. Während der Dosierung wird mit einem Eiswasserbad gekühlt. Die Innentemperatur steigt vorübergehend auf 66,8°C an.

### Variante II:

Es wird nicht die gesamte organische Phase aus der Vermischung von Formaldehyd-Lösung mit Anilin vorgelegt, sondern nur die Hälfte davon. Die andere Hälfte wird gleichzeitig mit der Salzsäure zugetropft.

### Variante III:

Es wird wie in Variante II vorgegangen. Statt mit einem Eiswasserbad wird die Reaktionsmischung aber durch Siedekühlung bei einem Druck von 60 - 80 mbar gekühlt. Die Innentemperatur steigt zu keinem Zeitpunkt über 50,0°C an.

### Beispiel 4 (erfindungsgemäßes Beispiel):

### Aminal Semi-Batch: Zulauf Aminal zu vorgelegtem Aniliniumhydrochlorid

### Variante I:

Es werden 3/4 des Anilins und die Formaldehyd-Lösung unter Rühren miteinander vermischt. Dabei entstehen zwei Phasen, die voneinander getrennt werden. Die organische Phase (= Aminal) wird in eine temperierte Pumpenvorlage überführt und auf 80°C temperiert.

In der Versuchsapparatur wird ¼ des Anilins (25°C) vorgelegt. Dazu wird unter Rühren die Salzsäure (25°C) zugegeben. Dabei entsteht eine klare Lösung (= Aniliniumhydrochlorid). Die Temperatur steigt vorübergehend auf ca. 70°C an. Es wird auf 50°C temperiert. Dann wird innerhalb von 10 min unter Rühren das Aminal zugepumpt und anschließend noch 5 min nachgerührt. Während der Dosierung wird mit einem Eiswasserbad gekühlt. Die Innentemperatur steigt vorübergehend auf 50,8°C an.

### Variante II: Die Dosierzeit beträgt 30 min

### Variante III: Die Dosierzeit beträgt 60 min

### Beispiel 5 (erfindungsgemäßes Beispiel):

### Aminal Semi-Batch: Simultanzulauf Aminal + Anilinium-HCl

Es wird wie in Beispiel 4 verfahren, jedoch wird nur die Hälfte der hergestellten Aniliniumhydrochlorid-Lösung (d.h. insgesamt nur die Hälfte von ¼ des Anilins) in der Vorlage belassen. Die andere Hälfte wird in einen Tropftrichter überführt und gleichzeitig mit dem Aminal zur Reaktionsmischung dosiert.

Die Ergebnisse sind in Tabelle 1 gezeigt.

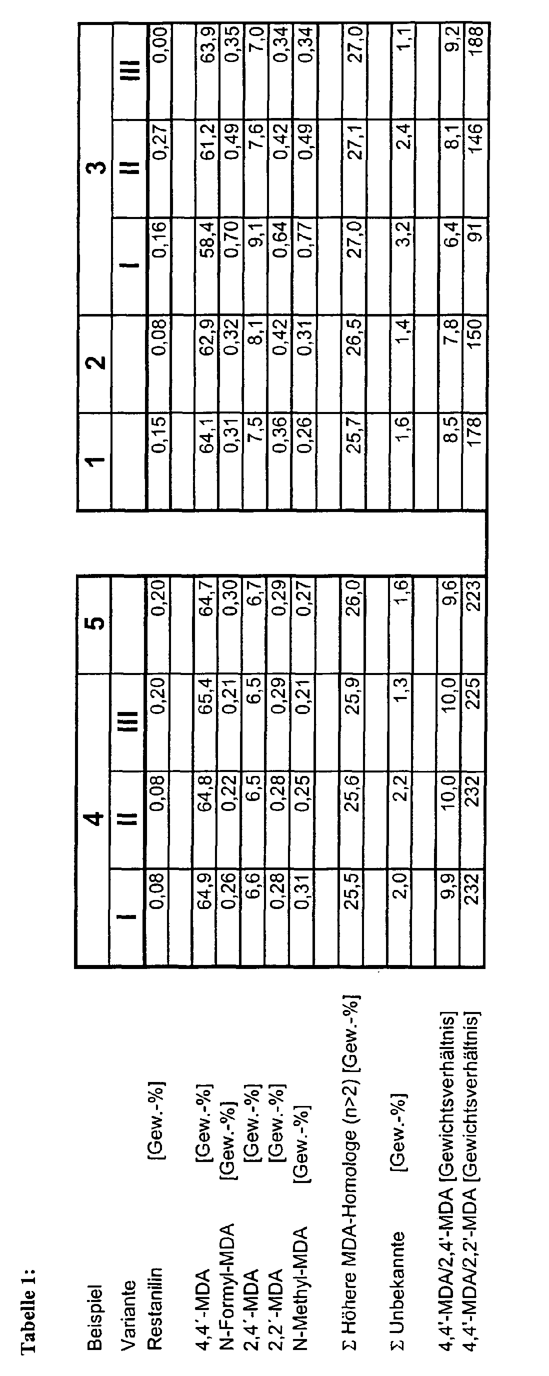

Die Versuchsergebnisse zeigen die Vorteile des erfindungsgemäßen Verfahrens auf:
- höherer Gehalt an monomerem 4,4'-MDA im Endprodukt
- höhere para-Selektivität (gemessen als Gewichtsverhältnis 4,4'-MDA : 2,4`-MDA sowie als Gewichtserhältnis 4,4'-MDA : 2,2'-MDA; mehrpara-Isomer ist vorteilhaft)

Durch die Erfindung werden Überkonzentrationen und Temperaturspitzen am Reaktionsort wirkungsvoll verhindert, ohne dass besondere, aufwendige technische Einrichtungen notwendig sind.

## Patentansprüche

1. Verfahren zur Herstellung von Di- und Polyaminen der Diphenylmethanreihe, bei dem man
a) eine erste Teilmenge an Anilin mit einem sauren Katalysator vermischt, und
b) eine zweite Teilmenge an Anilin mit Formaldehyd vermischt, und
c) die in den Schritt a) und b) hergestellten Mischungen anschließend miteinander vermischt und zur Reaktion bringt.

2. Verfahren nach Anspruch 1, bei dem man aus der in Schritt b) hergestellten Mischung zunächst die wässrige Phase teilweise oder vollständig abtrennt und die verbleibende Mischung anschließend in Schritt c) mit der in Schritt a) hergestellten Mischung vermischt.

3. Verfahren nach Anspruch 1 oder 2, bei dem man nach der Vermischung in Schritt c) der entstandenen Reaktionsmischung eine zusätzliche Menge an Formaldehyd zugibt.

4. Verfahren nach einem der Ansprüche 1 bis 3, bei dem man die Vermischung in Schritt c) halbkontinuierlich durchführt.

5. Verfahren nach einem der Ansprüche 1 bis 4, bei dem man als sauren Katalysator in Schritt a) Salzsäure einsetzt.

6. Verfahren nach einem der Ansprüche 1 bis 5, bei dem man in Schritt c) die Vermischung und/oder die Reaktion in Apparaten von größer 3 m³ Volumen durchführt.

7. Verfahren nach einem der Ansprüche 1 bis 6, bei dem die gesamte erforderliche Kühlleistung für die Schritte a), b) und c) größer 500 kW beträgt.

8. Verfahren zur Herstellung von Di - und Polyisocyanaten der Diphenylmethanreihe, bei dem man
a) eine erste Teilmenge an Anilin mit einem sauren Katalysator vermischt, und
b) eine zweite Teilmenge an Anilin mit Formaldehyd vermischt, und
c) die in den Schritt a) und b) hergestellten Mischungen anschließend miteinander vermischt und zu Di- und Polyaminen der Diphenylmethanreihe umsetzt, und anschließend
d) die Di- und Polyamine der Diphenylmethanreihe mit Phosgen zu Di- und Polyisocyanaten der Diphenylmethanreihe umsetzt.
